# EUROPEAN PATENT SPECIFICATION

(11) **EP 1 532 239 B1**
(45) Date of publication and mention of the grant of the patent: **07.01.2009**
(21) Application number: 03788380.8
(22) Date of filing: 12.08.2003
(51) Int. Cl.: C12N 9/14, C12N 9/48, C12N 9/76, C12N 9/50, C12N 9/64, C12Q 1/34, C12Q 1/37, C12P 21/06, C07H 21/04, C07K 17/00, G01N 33/50

(54) **METHODS AND COMPOSITIONS FOR TARGETING SECRETORY LYSOSOMES**
VERFAHREN UND ZUSAMMENSETZUNGEN ZUM TARGETING SEKRETORISCHER LYSOSOMEN
METHODES ET COMPOSITIONS DE CIBLAGE DE LYSOSOMES SECRETOIRES

(30) Priority: 14.08.2002 US 403464 P
(43) Date of publication of application: 25.05.2005
(73) Proprietor: Boehringer Ingelheim Pharmaceuticals Inc., Ridgefield, Connecticut 06877-0368 (US)
(72) Inventor: RAJOTTE, Daniel, Ridgefield, CT 06877-0368 (US); KABCENELL, Alisa, Ridgefield, CT 06877-0368 (US)
(74) Representative: Mahlbacher, Volker
(86) International application number: PCT/US2003/025098
(87) International publication number: WO 2004/016212

(56) References cited:
- MIESENBÖCK ET AL: "Visualizing secretion and synaptic transmission with pH-sensitive Green Fluorescent Proteins" NATURE, NATURE PUBLISHING GROUP, LONDON, GB, vol. 394, 9 July 1998 (1998-07-09), pages 192-195, XP002083568 ISSN: 0028-0836
- EL MESKINI RAJAA ET AL: "A signal sequence is sufficient for green fluorescent protein to be routed to regulated secretory granules" ENDOCRINOLOGY, vol. 142, no. 2, February 2001 (2001-02), pages 864-873, XP002431892 ISSN: 0013-7227
- HAUBENSAK WULF ET AL: "BNDF-GFP containing secretory granules are localized in the vicinity of synaptic junctions of cultured cortical neurons" JOURNAL OF CELL SCIENCE, vol. 111, no. 11, June 1998 (1998-06), pages 1483-1493, XP002431893 ISSN: 0021-9533
- LANG T ET AL: "CA2+-TRIGGERED PEPTIDE SECRTION NEUROTECHNIQUE IN SINGLE CELLS IMAGED WITH GREEN FLUORESCENT PROTEIN AND EVANESCENT-WAVE MICROSCOPY" NEURON, CAMBRIDGE, MA, US, vol. 18, no. 6, June 1997 (1997-06), pages 857-863, XP008019140
- MASUDA ET AL: 'Rab37 is a novel mast cell specific GTPase localized to secretory granules' FEBS LETTERS vol. 470, 2000, pages 61 - 64, XP002944826
- HALLGREN J. ET AL: 'MECHANISM FOR ACTIVATION OF MOUSE MAST CELL TRYPTASE: DEPENDENCE ON HEPARIN AND ACIDIC PH FOR FORMATION OF ACTIVE TETRAMERS OF MOUSE MAST CELL PROTEASE 6' BIOCHEMISTRY vol. 39, no. 42, 24 October 2000, pages 13068 - 13077, XP001088517

## Description

### BACKGROUND

This invention relates to methods and compositions for targeting proteins to secretory lysosomes. The invention further provides methods of use in drug screening assays, and methods of purifying secretory lysosomes.

Mast cells are specialized secretory cells that release a variety of biologically active substances. Mast cells are found resident in tissues throughout the body, particularly in association with structures such as blood vessels, nerves, and in proximity to surfaces in contact with the external environment (see Metcalfe et al. Physiol Rev. 77:1033-1079, 1997). Mast cell activation may be initiated upon interaction of a multivalent antigen with its specific IgE antibody attached to the cell membrane via its high affinity receptor, FcεRI. Mast cells and basophils play a central role in inflammatory and allergic reactions (see Williams, et al. J. Allergy Clin. Immunol. 105:847-859, 2000). They are known to release potent inflammatory mediators such as histamine, proteases, chemotactic factors and metabolites of arachidonic acid that act on the vasculature, smooth muscle, connective tissue, mucous glands and inflammatory cells.

The pathways for granule biogenesis and exocytosis in mast cells are still largely obscure (Griffiths, G.M. et al., Biochem Biopys Res Commun. 222(3): 802-808, 1996; Masuda et al., FEBS Lett. 470:61-64, 2000; Baram, D. et al., J Immunol. 167(7):4008-4016, 2001). Mast cells contain structures known as secretory lysosomes which are a mixture of lysosomes and secretory granules (Stinchcombe and Griffiths, J Cell Biol. 146(1):1-6, 1999). The mast cell granule can be described as a modified lysosome, specialized for fusion with the plasma membrane, and with other lysosomal granules, after receptor activation. Although similar secretory lysosomes are found in hematopoietic cells, little is known about the mechanisms by which these organelles receive and deliver their cargo. For example, Riesbeck et al. (WO 98/42850) disclose protein targeting to endothelial cell Weibel-Palade bodies. Weibel-Palade bodies contain the adhesion molecule P-selectin.

There are two categories of inflammatory mediators in mast cells and basophils, preformed mediators and newly formed mediators. Preformed mediators, stored in the cytoplasmic granules of rodent or human mast cells, include histamine, proteoglycans, cytokines, serine proteases, carboxypeptidase A and small amounts of sulfatases and exoglycosidases (Metcalfe et al., Physiol Rev. 77(4):1033-1079, 1997). Histamine acts on a set of receptors (H1, H2, H3, H4) on cells and tissues and is rapidly metabolized extracellularly. Proteoglycans may function to package histamine and basic proteins into secretory granules, and in human mast cells may stabilize the protease tryptase. Neutral proteases, which account for the vast majority of the granule protein, serve as markers of mast cells. Newly generated mediators, often absent in resting mast cells, consist of arachidonic acid metabolites, principally leukotriene C and prostaglandin D. These mediators are typically produced during IgE receptor activation. Of particular interest in humans is the production of tumor necrosis factor (TNF), Interleukin (IL)-4, IL-5 and IL-6.

Proteases are the major protein constituent exocytosed from activated mast cells (Huang et al., J Clin Immunol. 18:169-183, 1998). Tryptases, chymases, and carboxypeptidases are the three major families of proteases stored in the secretory granules of mast cells. Chymases are part of the serine protease family. Immunohistochemical localization indicates that they are only synthesized in mast cells (Beil et al., Histol Histopathol. 15(3):937-946, 2000). Human, primate, and dog chymase generate angiotensin II (Ang II) from Ang I, while mouse and rat chymases degrade Ang II (Fukami et al., Curr Pharm Des. 4(6):439-453, 1998). Chymase also degrades extracellular matrix, and processes procollagenase, inflammatory cytokines and other bioactive peptides. As a result, chymase plays important roles in inflamed tissues through its proteolytic activities.

In human cells, genes encoding two chymotryptic enzymes (chymase and Cathepsin G-like protease) and one mast cell carboxypeptidase enzyme and at least two genes encoding tryptase peptides have been detected. The gene encoding chymase is closely linked to the gene encoding cathepsin G, an enzyme apparently expressed in mast cells, and to the genes encoding granzymes. Mucosal (MC)-type mast cells contain tryptase, chymase, cathepsin G-like protease and mast-cell carboxypeptidase. The biological function of mast cell neutral proteases, like mast cells themselves, remains to be fully clarified. For example, on-going mast cell activation in asthma appears to be a characteristic of this chronic inflammatory disease.

In murine mast cells, five chymases (Mouse Mast Cell Protease (MMCP)-1, -2, -3, -4, and -5), one mast cell carboxypeptidase and two tryptases (MMCP-6 and -7) have been reported. In rodents, the protease composition of mast cell subsets differs. In rats two isoforms of chymase, Rat Mast Cell Protease (RMCP) I (Lagunoff and Pritzl Arch Biochem Biophys. 173(2):554-563,1976) and RMCP II (Kido et al, Arch Biochem Biophys. 239(2):436-443, 1985) were found to distinguish the mast cells in mucosal surfaces (RMCP-II positive), from other mast cells (RMCP-I positive) (Gibson and Miller Immunology 58(1):101-104, 1986). More recently, two additional serine proteases were isolated by PCR amplification from rat serosal MC: the rat tryptase (the counterpart of MMCP-6) and an additional chymase named RMCP III (Lutzelschwab et al., J Exp Med. 185(1):13-29, 1997). The latter protease is the rat counterpart of mouse MMCP-5.

Scientists have reported on a protein, Rab37, that can localize to the surface of mast cell granules when fused to green fluorescent protein (GFP) and is over-expressed in bone-marrow derived mast cells (see Masuda et al., FEBS Lett. 470:61-64, 2000). Rab37 appears to localize to the cytoplasmic surface of granules. However, Masuda does not teach the use of Rab37 to target granules.

Until the present invention, there have been no reports on the use of a targeting moiety to localize proteins to secretory lysosomes. Current state of the art employs indirect methods for the detection of granule content or exocytotic activity. For example, mast cell granules have been studied by monitoring their content with antibodies, or measuring the activity of enzymes such as hexosaminidase (see Schwartz et al. J. Immunol. 123:1445-1450, 1979; and Dragonetti et al. J. Cell Sci. 113:3289-3298, 2000). Other indirect methods relate proteins and their functions to other secretory compartments such as the endoplasmic reticulum, Golgi and trans Golgi network (see Donaldson and Lippincott-Schwartz, Cell 101:693-696, 2000). Therefore, a targeting moiety localizing proteins to the inner core of mast cell secretory lysosomes is a significant advancement in mast cell research and drug discovery.

Current methods that attempt to quantify the release of mediators upon degranulation of cells containing secretory lysosomes are lengthy and costly (see, for example, Schwartz et al., J Immunol. 123:1445-1450, 1979 and Schulman et al., J Immunol. 131:2936-1941, 1983). The present invention overcomes these obstacles such that not only is the content of secretory lysosomes quantified, but also the movement of secretory lysosomes is monitored in real time.

Until the present invention, no methods were amenable for High Throughput Screening (HTS) to screen for modulators of secretory lysosomes. Current state of the art does not allow direct monitoring of cell degranulation in an HTS setting. For example Demo et al. (Cytometry, 36(4):340-8, 1999) disclose assays that require several biochemical measurements and many steps that consume time, energy and likely have low reproducibility due to the reagents used (histamine , tryptase, hexosaminidase). For example, histamine is known to have a poor dynamic range for quantification in HTS setting. Other methods have been used to monitor cell degranulation using fluorescent probes such as acridine orange (see Love Histochemistry 62:221-225, 1979) but these assays lack specificity for the exocytotic process and poor-signal to noise ratios (see Demo et al. Cytometry, 36(4):340-8, 1999). One group has disclosed a quantitative measurement of cell degranulation using a flow cytometric annexin-V binding assay (see Demo et al. Cytometry, 36(4):340-8, 1999). Flow cytometry however, is not amenable to HTS format.

Current art provides protocols to obtain subcellular fractions enriched in mast cell granules by fractionation of cell homogenates on Percoll or sucrose gradients (Lindmark et al., J Leukocyte Biol. 66:634-643, 1994). Advantageously, the present invention provides methods to purify secretory lysosomes to a higher degree than currently achieved. The increased level of purification achieved by the present invention is crucial for proteomics studies aimed at the identification of novel drug discovery targets involved in cell activation.

pH-sensitive mutants of green fluorescent protein (so called pHluorins) have been described as tools to monitor secretory vesicle exocytosis and recycling in neural cells (Miesenböck, G. et al., Nature 394: 192-195, 1998). When linked to a vesicle membrane protein such pHluorins were sorted to secretory and synaptic vesicles.

An artificial fusion protein containing mouse mast cell protease 6 (mMCP-6) linked via an N-terminal 6 x His tag to an enterokinase (EK) site replacing its native activation peptide has been described by Hallgren, J. et al., Biochemistry 39:13068-13077, 2000. This mouse mast cell tryptase is naturally stored in mast cell secretory granules in complex with heparin proteoglycan. During allergic inflammation these complexes are released via mast cell degranulation. As the result of Hallgren's studies it has been found that this tryptase is activated naturally through a proteolytic cleavage by heparin at an acidic pH after exocytosis.

### BRIEF SUMMARY

The present invention relates to methods for localizing secretory lysosomes in real-time within a cell using a targeted molecule. Such methods are useful for many purposes, including but not limited to, studying secretory lysosome movement and fusion, directly monitoring exocytosis, developing cellular screens for cell activation and purification of secretory lysosomes.

Prior to the present invention, there were no existing direct methods of targeting protein to secretory lysosomes. Mast cell secretory lysosomes are specialized organelles that contain proteases, heparin, histamine and several cytokines. Previously, secretory lysosomes were studied by indirect methods such as monitoring their content with antibodies or measuring the activity of enzymes such as hexosaminidase.

The present invention relates to a mast cell, basophil, hemopoietic cell, melanocyte or goblet cell secretory lysosome targeting fusion moiety comprising: (a) a polypeptide that specifically localizes to a secretory lysosome and is released with the granule content into the extracellular milieu after stimulation and degranulation of the cell and (b) a fluorescent label polypeptide. The present invention also relates to a mast cell, basophil, hemopoietic cell, melanocyte or goblet cell secretory lysosome targeting fusion moiety comprising: (a) a nucleotide sequence encoding a polypeptide that specifically localizes to a secretory lysosome and is released with the granule content into the extracellular milieu after stimulation and degranulation of the cell and (b) a nucleotide sequence encoding a fluorescent label polypeptide.

The present invention further relates to a secretory lysosome targeting fusion moiety as defined above comprising a protease selected from the group consisting of tryptases, chymases, and carboxypeptidases, preferably Mouse Mast Cell Protease 1, -2, -3. -4, -5, -6, and -7; Rat Mast Cell Protease I and Rat Mast Cell Protease Π; human chymases; human tryptases; Cathepsin G-like protease; Cathepsin G; carboxypeptidase A, and hexosaminidase; more preferably Rat Mast Cell Protease II according to Gen Bank accession no. J02712.

The invention further relates to a cell comprising a mast cell, basophil, hemopoietic cell, melanocyte or goblet cell secretory lysosome targeting fusion moiety of the present invention.

In a specific embodiment, the fluorescent molecule (fluorescent label polypeptide) is *Discosoma* sp. red fluorescent protein or green fluorescent protein. In an embodiment, the cell of the invention is selected from the group consisting of : mast cells, basophils, hemopoietic cells, melanocytes, and goblet cells. In a specific embodiment, the cell is a mast cell.

In a particular embodiment, the invention relates to a cell line expressing a mast cell basophil, hemopoietic cell, melanocyte or goblet cell secretory lysosome targeting fusion moiety as deposited with the American Type Culture Collection and assigned accession number PTA-4571.

In one embodiment of the present invention, there is disclosed a method for detecting and quantifying degranulation comprising: (a) incubating a cell expressing a mast cell, basophil, hemopoietic cell, melanocyte or goblet cell secretory lysosome targeting fusion moiety according to the invention in the presence of a cell activator; (b) incubating the cell expressing the secretory lysosome targeting fusion moiety in the absence of the cell activator; and (c) detecting and quantifying the release of label in the supernatant in the presence of the cell activator compared to the release of label in the supernatant in the absence of the cell activator, wherein an increase in the release of label in the supernatant in the presence of the cell activator indicates degranulation.

In another embodiment of the present invention, there is disclosed a method for detecting and quantifying inhibition of degranulation comprising: (a) incubating a cell expressing a mast cell, basophil, hemopoietic cell, melanocyte or goblet cell secretory lysosome targeting fusion moiety according to the invention with a cell or activator in the presence of a test substance; (b) incubating the cell expressing the secretory lysosome targeting fusion moiety with the cell activator in the absence of the test substance; and (c) detecting and quantifying a change in the release of label in the supernatant in the presence of the test substance compared to the release of label in the supernatant in the absence of the test substance, wherein a decrease in the release of label in the supernatant in the presence of test substance indicates inhibition of degranulation.

In another embodiment of the present invention, there is disclosed a method for detecting and quantifying degranulation at the single cell level comprising: (a) incubating a cell expressing a mast cell, basophil, hemopoietic cell, melanocyte or goblet cell secretory lysosome targeting fusion moiety according to the invention in the absence of a cell activator, (b) detecting and quantifying the amount of label in the absence of a cell activator, (c) incubating the cell of step (a) in the presence of a cell activator; (d) detecting and quantifying the amount of label in the presence of the cell activator; and (e) detecting a change in the amount of label in the cell in the presence of the cell activator compared to the amount of label in the cell in the absence of the cell activator, wherein a decrease in the amount of label indicates degranulation.

In another embodiment of the present invention, there is disclosed a method for detecting and quantifying degranulation at the single cell level comprising: (a) incubating a cell expressing a mast cell, basophil, hemopoietic cell, melanocyte or goblet cell secretory lysosome targeting fusion moiety according to the invention in the presence of a cell activator; (b) detecting and quantifying the amount of label in the presence of the cell activator, (c) incubating the cell of step (a) in the presence of the cell activator and a test substance; (d) detecting and quantifying the amount of label in the presence of the cell activator and the test substance; and (e) comparing the amount of label in the cell in the presence of the test substance to the amount of label in the cell in the absence of the test substance, wherein an increase in the amount of label in the presence of the test compound indicates degranulation.

### BRIEF DESCRIPTION OF THE FIGURES

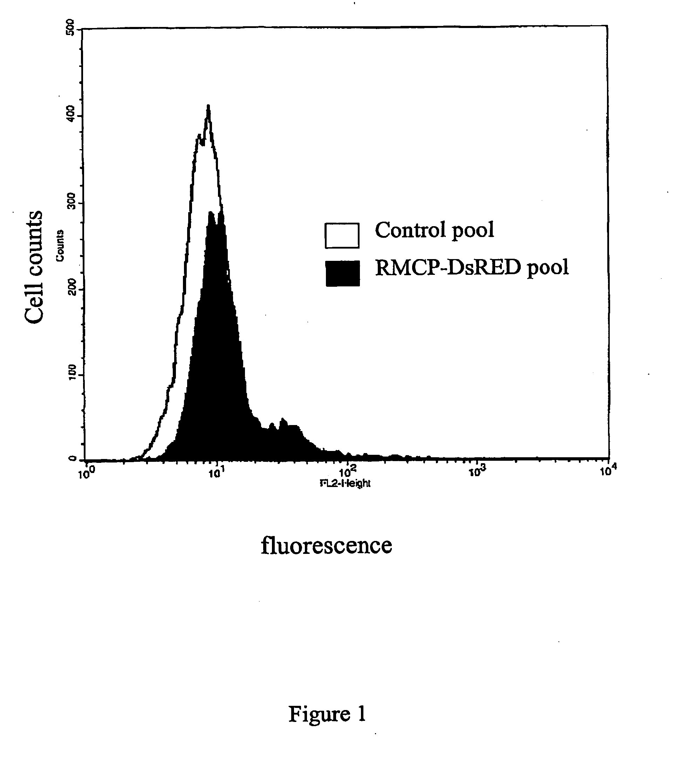

**Figure 1****. Stable expression of RMCP-DsREd in RBL-2H3** cells. Cells were transfected by electroporation with the RMCP-DsRED vector ("DsRED", *Discosoma sp.* red fluorescent protein). Cells were then submitted to Geneticin® (G-418, GIBCO Invitrogen Corp., Carlsbad, CA) selection for 10 days. Figure 1 shows FACS analysis of control RBL-2H3 population compared to the selected pool of cells expressing RMCP-DsRED.

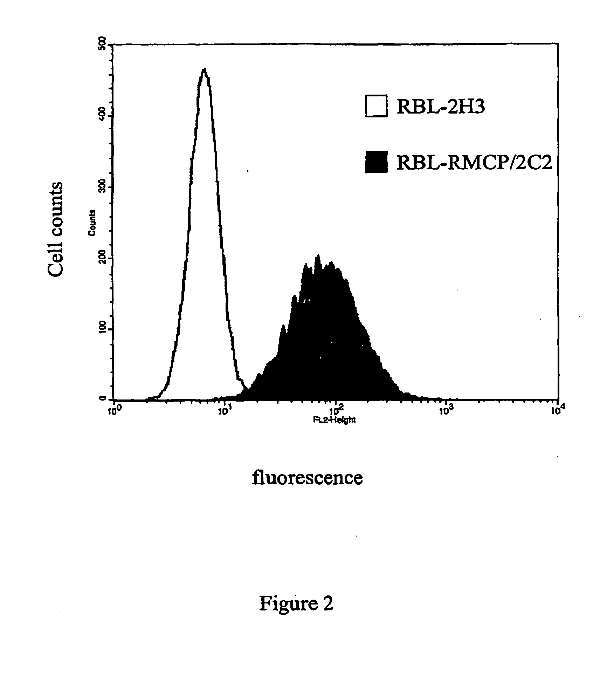

**Figure 2****. FACS analysis of the clone RBL-RMCP/2C2.** Individual clones from the cellular pool expressing RMCP-DsRED were generated. This figure shows FACS analysis of the RBL-2H3 parental cells compared to the clone RBL-RMCP/2C2.

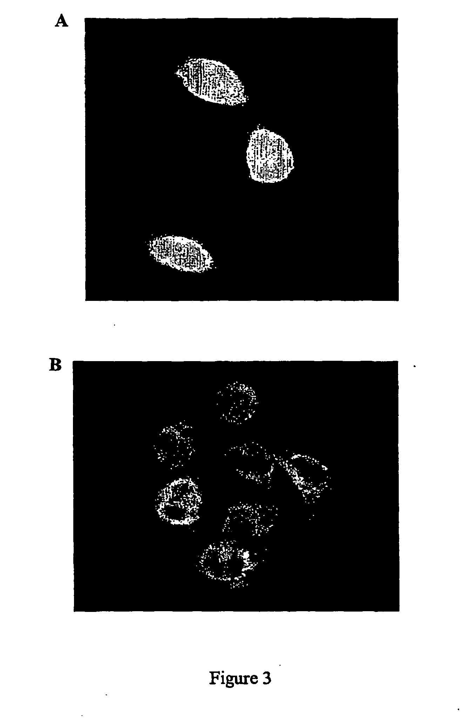

**Figure 3****. Stable expression of RMCP-DsRED recombinant protein in RBL-2H3 cells.** RBL-2H3 cells were stably transfected with an expression vector for the DsRED protein alone or the RMCP-DsRED fusion protein. After the selection process, individual clones were analyzed by confocal microscopy to determine subcellular distribution of the recombinant protein. A) Shows confocal image of cells expressing the DsRED protein and its cytoplasmic expression. B) Shows a confocal image of cells expressing the RMCP-DsRED fusion protein. The punctate fluorescence correlates with the localization of granules or secretory lysosomes in mast cells or basophils.

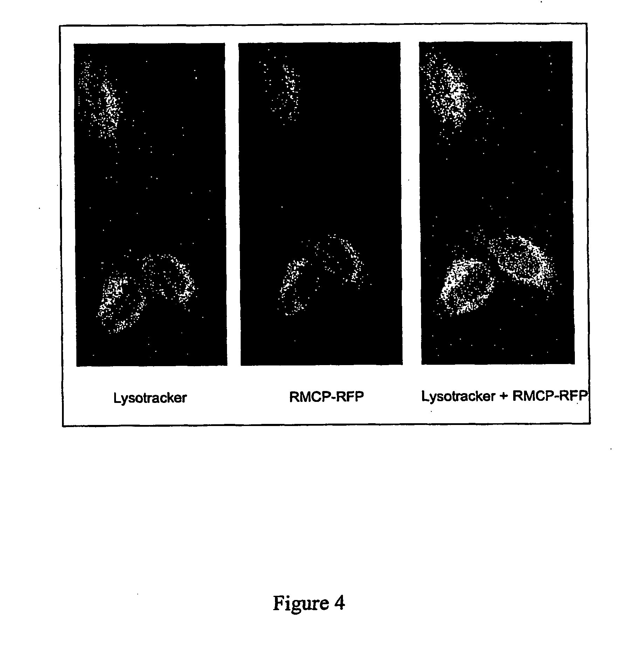

**Figure 4****. The RMCP-DsRED construct targets secretory lysosomes and granules in RBL 2H3 cells.** Cells stably expressing the RMCP-DsRED fusion protein were treated with the LysoTracker® probe. Left panel shows intracellular localization of secretory lysosomes and granules using the LysoTracker® probe. Middle panel shows the distribution of the RMCP-DsRED fusion protein. The overlay of LysoTracker® and RMCP-DsRED images (right panel) shows colocalization of the RMCP-DsRED with the secretory lysosome and granule compartments.

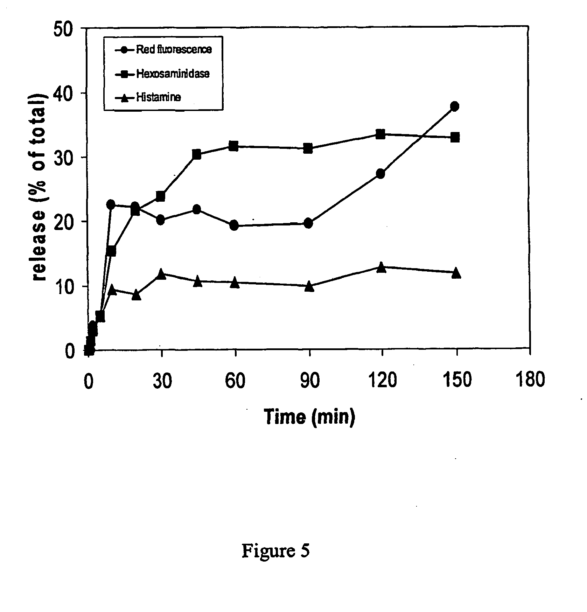

**Figure 5****. RMCP-DsRED is released upon IgE stimulation of RBL 2H3 cells. RBL 2H3** cells stably expressing the RMCP-DsRED protein (clone RBL-RMCP/2C2) were stimulated with IgE and antigen (DNP-HSA). Hexosaminidase and histamine are two granule markers that are released from the cells within minutes after stimulation. Quantification of the fluorescence released after stimulation shows a rapid release of the RMCP-DsRED fusion protein followed by a slower phase of release 90 minutes after stimulation.

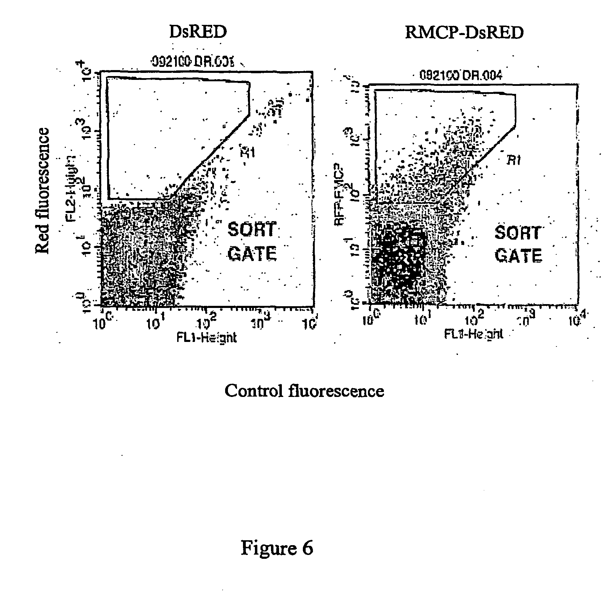

**Figure 6****. Flow cytometric analysis of fluorescent granules from RBL-2H3 clones stably expressing RMCP-DsRED protein.** Cells expressing DsRED alone (control) or RMCP-DsRED were submitted to subcellular fractionation on a Percoll gradient. The fraction containing the secretory lysosome and granules was then analyzed by organelle flow cytometry. Cells expressing DsRED (left panel) showed no fluorescent lysosomes or granules compared to cells expressing RMCP-DsRED (right panel).

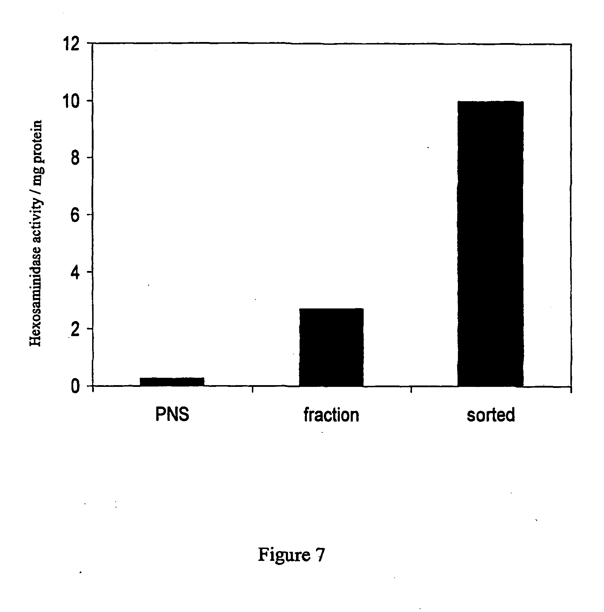

**Figure 7****. Purification of fluorescent secretory lysosomes and granules using FACS sorting.** Fluorescently labeled secretory lysosomes and granules gated in figure 6 (right panel) were sorted by flow cytometry and assayed for hexosaminidase content Hexosaminidase specific activity is shown for the post nuclear supernatant (PNS), the lysosomal/granule fraction isolated by Percoll gradient and for the FACS sorted material.

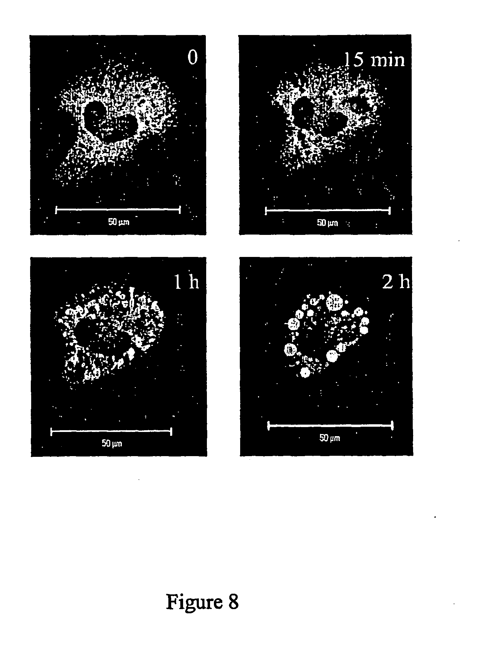

**Figure 8****. Live cell imaging of RBL-2H3 cells expressing RMCP-DsRED following IgE stimulation.** Live RBL-RMCP/2C2 cells were visualized by confocal microscopy. Cells were imaged at time 0 and then stimulated with IgE and antigen (DNP-HSA). Cells were incubated for a total of 2 hours and images were taken at various time points. The 15 min., 1h and 2h time points are shown.

### DETAILED DESCRIPTION OF THE INVENTION

### 1. Definitions

The following definitions are provided to facilitate understanding of certain terms used herein:

As used herein and in the appended claims, the singular forms "a", "an", and "the" include plural reference unless the context clearly dictates otherwise.

By "cells" it is meant to include cells in any form, including, but not limited to, cells retained in tissue, cell clusters and individually isolated cells.

By "cell line" it is meant cells capable of stable growth *in vitro* for many generations.

By "clone" it is meant a population of cells derived from a single cell or common ancestor by mitosis.

By "degranulation" it is meant movement and exocytosis of secretory lysosomes.
By "polypeptide" it is meant peptide or protein and variants thereof.

By "secretory lysosome" it is meant a dual-function organelle that is used as both the lysosome (for degradation) and for storage of secretory proteins of the cell and which shares many features with both conventional lysosomes and secretory granules, such as structure and content.

By "secretory lysosome targeting fusion moiety" it is meant a moiety comprising: (a) a polypeptide that specifically localizes to a secretory lysosome or a nucleotide sequence encoding such polypeptide and (b) a label polypeptide or nucleotide sequence encoding such label polypeptide.

By "secretory lysosome targeting moiety" it is meant a polypeptide that specifically localizes to a secretory lysosome or a nucleotide sequence encoding such polypeptide.

By "variant" it is meant a sequence, such as a polypeptide, that differs from another sequence, but retains essential properties thereof that is, properties for which the sequence is utilized in its application (e.g., protease activity). For example, a variant of a polypeptide may differ in amino acid sequence by one or more substitutions, additions, and deletions from the reference polypeptide. By "variant" it is also meant to include fragments of a full length sequence that retain essential properties thereof.

### 2. The Methods and Constructs

The present invention overcomes many of the problems associated with prior art methods for detection and monitoring of secretory lysosome content and exocytotic activity. By transfecting cells with a moiety comprising a nucleotide sequence encoding a secretory lysosome-specific protein and a nucleotide sequence encoding a fluorescent label polypeptide, the present invention permits the study of the movement of secretory lysosomes and the release of their contents in real time and secretory lysosome quantification in living cells.

Cells that may be used in the present invention include cell lines and primary cells that have secretory lysosomes, comprising mast cells, basophils, hemopoietic cells, melanocytes, and goblet cells. In a preferred embodiment, the cells are mast cells. In one embodiment, a cell line expressing a secretory lysosome targeting moiety is designated RBL-RMCP/2C2 (accession no. PTA-4571,deposited on August 7, 2002 with the American Type Culture Collection, 10801 University Boulevard, Manassas, VA 200110-2209 under the terms of the Budapest Treaty).

The secretory lysosome targeting fusion moiety of the present invention localizes in secretory lysosomes. The secretory lysosome targeting fusion moiety of the present invention may comprise constituents of secretory lysosomes such as proteases, for example, tryptases, chymases and carboxypeptidases including but not limited to: Mouse Mast Cell Protease Rat Mast Cell Protease -1, -2, -3.-4, -5, -6, and -7; Rat Mast Cell Protease Rat mast Cell Protease I and Rat Mast Cell Protease II; human chymases; human tryptases; Cathepsin G-like protease; Cathepsin G; carboxypeptidase A; hexosaminidase. In an embodiment, the secretory lysosome targeting moiety comprises Rat Mast Cell Protease II.

The secretory lysosome targeting fusion moiety of the present invention can further comprise any polypeptide of interest or nucleotide sequence encoding such polypeptide. In the screening and detection methods of the present invention, the polypeptide or nucleotide sequence encoding such polypeptide is a label, preferably a fluorescent molecule, e.g., *Discosoma sp.* red fluorescent protein (DsRED) or green fluorescent protein (GFP).

A fluorescent label polypeptide can include, but is not limited to, a luminescent molecule (*e.g*. luciferase), an enzyme (e.g. horse radish peroxidase, β-galactosidase), a fluorescent polypeptide (*e.g., Discosoma sp.* red fluorescent protein (DsRED) or green fluorescent protein (GFP).

In the therapeutic methods of the invention described below, the secretory lysosome targeting fusion moiety can further comprise any therapeutic polypeptide or nucleotide sequence encoding such polypeptide of interest, including but not limited to enzymes, cytokines, growth factors, and recombinant antibodies (single chains).

The present invention also provides methods for identifying compounds that modulate degranulation using the secretory lysosome targeting fusion moieties of the invention. For example, a cell of the invention expressing a mast cell, basophil, hemopoietic cell, melanocyte or goblet cell secretory lysosome targeting fusion moiety comprising a label polypeptide is incubated with a cell activator in the presence and absence of a test substance. A change in the release of fluorescence in the supernatant in the presence of the test substance would indicate that the test substance modulates degranulation, for example, an increase in the release of fluorescence in the supernatant indicates degranulation. In a preferred embodiment, the secretory lysosome targeting fusion moiety comprises a fluorescent label polypeptide.

Cell activators include but are not limited to: IgE and a multivalent antigen, phorbol myristate acetate, ionomycin, toll-like receptors, and protease receptors. In one embodiment of the invention cell activators are selected from the group consisting of: IgE and a multivalent antigen, phorbol myristate acetate, and ionomycin.

In another embodiment, the present invention provides methods for increasing the purity of secretory lysosome preparations using the secretory lysosome targeting fusion moieties of the invention. Preferably, a cell line transfected with a secretory lysosome targeting fusion moiety of the invention comprising a fluorescent polypeptide is fractionated by methods known in the art (*e*.*g*., Percoll or sucrose gradient) to obtain subcellular fractions enriched in secretory lysosome. The secretory lysosome -rich fraction is then further purified using fluorescence activated cell sorting (FACS).

In another embodiment, the present invention provides methods for studying secretory lysosome maturation, biosynthesis, cell differentiation, migration and activation *in vivo* using a secretory lysosome targeting moiety according to the invention further comprising a reporter gene.

In another embodiment, the present invention provides methods for studying and quantifying exocytosis (degranulation) in real time, at a single cell level using a secretory lysosome targeting fusion moiety according to the invention, preferably comprising a fluorescent label polypeptide, wherein detection and quantification is performed using fluorescence or confocal microscopy, for example, using a Cellomics ArrayScan® System (Cellomics, Inc., Pittsburgh, PA). In these methods, for example, fluorescence would be detected before and after stimulation of a cell transfected with a fluorescent marker and the secretory lysosome targeting moiety, wherein a reduction in fluorescence indicates degranulation. These methods, therefore can be used to screen for compounds that inhibit the release of secretory lysosomes.

In another embodiment, the present invention provides methods for delivery of therapeutic polypeptides *in vivo* using a secretory lysosome targeting fusion moiety according to the invention comprising a therapeutic polypeptide. In one embodiment, a cell line transfected with the therapeutic secretory lysosome targeting fusion moiety is encased in an immunoisolation device and implanted in a subject.

The screening methods of the present invention may be adapted to High Throughput Screening (HTS) and Ultra High Throughput Screening (UHTS). The HTS and UHTS can employ, for example, a Zymark Allegro™ modular robotic system (Zymark Corp., Hopkinton, MA) to dispense reagents, buffers, and test compounds into either 96-well or 384-well black microtiter plates (from Dynex (Dynex Technologies, Denkendorf, Germany) or Corning (Corning Costar, Cambridge, MA), respectively).

### EXAMPLES

The following examples are provided to illustrate the invention, but not to limit its scope. Other variants of the invention will be readily apparent to one of ordinary skill in the art.

### Example 1: Cloning of RMCP II in the expression vector pDsRED1-N1

The sequence encoding RMCP II was retrieved from GenBank (accession no. J02712; Benfey et al. JBC 262:5377, 1987). RMCP II was cloned by polymerase chain reaction (PCR). The rat basophilic leukemia (RBL-2H3) cell line (accession no. CRL-2256, American Type Culture Collection (ATCC), Manassas, VA) was used as a source of RNA. The reverse transcriptase reaction (first strand cDNA synthesis) was done using the Superscript^{™} amplification system by GIBCO BRL (catalog no. 18089-011, GIBCO BRL, Invitrogen Corp., Carlsbad, CA) according to the manufacturer's instructions. The PCR amplification was performed on first strand cDNA (from previous step) using the following oligos:
5'-TCAGATCTCGAGATGCAGGCCCTACTATTCCTG-3' (SEQ ID NO:1) and
5'-CTGCAGAATTCGGCTACTTGTATTAATGACTGCAT-3' (SEQ ID NO:2).

The PCR conditions were as follows: 94°C (30 sec) - 62°C (30 sec) - 72°C (50 sec). The PCR product was purified and digested with the restriction enzymes XhoI and EcoRI. These restriction sites are provided by the oligos. The fragment was then cloned in the same sites of the pDsRED1-N1 vector from Clontech (catalog no. 6921-1, BD Biosciences Clontech, Palo Alto, CA). This cloning strategy results in the full length RMCP II cDNA in-frame with the N-terminus of the cDNA for the fluorescent protein DsRED. The sequence identity of the recombinant vector, RMCP-DsRED, was confirmed by DNA sequencing.

### Example 2: Stable expression of RMCP-DsRED recombinant protein in RBL-2H3 cells

RBL-2H3 cells (8x10⁶) were transfected with the RMCP-DsRED vector (45 µg) by electroporation (Guillemot et al. JCB 110:2215-2225, 1997). The conditions for the electroporation were 300 V and 960 µF in a volume of 800 µl. The transfected cell line is designated RBL-RMCP/2C2. Transfected cells were transferred to the appropriate culture media and incubated for 48 h. Positive clones were then selected by the addition of 1 mg/ml of active Geneticin® (GIBCO BRL, Invitrogen Corp., Carlsbad, CA). Ten days after transfection, the cells were analyzed by fluorescence activated cell sorting (FACS). As seen in Figure 1, a population of cells positive for red fluorescence was detected. Individual clones from the cell population were isolated by FACS and amplified. As seen in Figure 2, RBL-RMCP/2C2 is a positive clone expressing the RMCP-DsRED fusion protein.

### Example 3: Expression of RMCP-DsRED fusion protein in granules

The subcellular localization of the RMCP-DsRED protein from the RBL-RMCP/2C2 clone was analyzed by confocal microscopy. A cellular clone expressing the pDsRED vector alone was used as control. As shown in Figure 3, cells transfected with the pDsRED control vector expressed the DsRED protein in the cytoplasm (diffuse fluorescence). In contrast, RMCP-DsRED fusion shows punctuate expression in proximity to the plasma membrane. This pattern correlates with the localization of granules in mast cells or basophils. The LysoTracker® probe (Molecular Probes, catalog no. L-7526) is a weakly basic amine that selectively accumulates in cellular compartments with low internal pH which include lysosomes and dense core granules (Sreyer, JA et al. Nature 388:474-478, 1997). As seen in Figure 4, in the RBL-RMCP/2C2 clone, the LysoTracker® probe co-localizes with the RMCP-DsRED fusion protein. This result confirmed that the RMCP-DsRED fusion protein is targeted to granules.

### Example 4: Release of red fluorescence upon cell activation

Mast cells and basophils respond to IgE and antigen stimulation by the rapid release (within minutes) of their granule content into the extracellular milieu. A standard assay in the field is to stimulate the RBL-2H3 cell line with an antigen-specific IgE molecule and then cross-link the IgE receptor by the addition of the corresponding antigen (Roa M. et al. J. Immunol. 159:2815-2823, 1997).. The release of histamine and β-hexosaminidase are typically used as markers to monitor degranulation (Schwartz LB et al. J. Immunol. 123:1445-1450, 1979). As expected, when the RBL-RMCP/2C2 cells are stimulated with a mouse anti-DNP (dinitrophenyl) IgE followed by stimulation with DNP-HSA (DNP antigen coupled to human serum albumin) they release both histamine and β-hexosaminidase within minutes (Figure 5). As shown in Figure 5, the RBL-RMCP/2C2 cells also release red fluorescence upon stimulation. The fluorescence release was measured from the supernatant of the cell culture (in a 96-well plate format) at the indicated time using an LJL fluorescence plate reader. The kinetics of histamine, β-hexosaminidase and fluorescence release are the same.

### Example 5: Purification of granules by FACS

Cell homogenates were fractionated on Percoll or sucrose gradients to obtain subcellular fractions enriched in mast cell granules (Kruger P.G. et al., Exp. Cell Res. 129:83-93, 1980). Using the RBL-RMCP/2C2 cell line, the granule-rich fraction was further purified by FACS. As shown in Figure 6, a distinct fluorescent population was detected in the granule fraction isolated from RBL-RMCP/2C2 cells as compared to a control cell line. The positive (gated) population was then separated from the total fraction by organelle sorting (Fialka I. et al. J. Biol. Chem. 274:26233-26239, 1999). As shown in Figure 7, hexosaminidase specific activity was substantially increased after sorting. Thus, this step contributed to a.substantial increase in the purity of the granule fraction.

### Example 6: Live imaging of cells expressing RMCP-DsRED following IgE and antigen stimulation

Live RBL-RMCP/2C2 cells were visualized by confocal microscopy. Cells were sensitized with anti-DNP IgE, imaged at time 0 and then stimulated with antigen (DNP-HSA). Cells were incubated for a total of 2 hours and images were taken at various time points. The 15 min, 1h and 2h time points are shown in Figure 8. As shown in Figure 8, degranulation can be observed in real time at the single cell level.

### Example 7: Quantifying the inhibition of degranulation by a test substance in RBL-RMCP/2C2 cells

Cells are seeded in 96-well plates at a density of 2X10⁴ cells per well and incubated overnight. Cells are then washed twice in culture media and incubated for 2 hours at 37°C in culture media containing the test substance (concentrations ranging from 1 mM to 1 pM) and 1 µg/ml of anti-DNP IgE monoclonal antibody (SPE7 clone, Sigma). The cells are then washed twice in Tyrode's buffer (10 mM Hepes, pH 7.4, 130 mM NaCl, 5 mM KCl, 1.4 mM CaCl₂, 1 mM MgCl₂, 5.6 mM glucose, and 0.1% BSA) and then stimulated with 100 ng/ml DNP-HSA (Sigma) in Tyrode's buffer for one hour. Aliquots (100 µl) from the culture supernatants are analyzed for release of red fluorescence. The red fluorescence is detected on an LJL Bioanalyst (LJL Biosystems, Sunnyvale,CA) set at 530 nm for excitation and 580 nm for emission.

## Claims

1. A mast cell, basophil, hemopoietic cell, melanocyte or goblet cell secretory lysosome targeting fusion moiety comprising: (a) a polypeptide that specifically localizes to a secretory lysosome and is released with the granule content into the extracellular milieu after stimulation and degranulation of the cell and (b) a fluorescent label polypeptide.

2. A mast cell, basophil, hemopoietic cell, melanocyte or goblet cell secretory lysosome targeting fusion moiety comprising: (a) a nucleotide sequence encoding a polypeptide that specifically localizes to a secretory lysosome and is released with the granule content into the extracellular milieu after stimulation and degranulation of the cell and (b) a nucleotide sequence encoding a fluorescent label polypeptide.

3. A mast cell, basophil, hemopoietic cell, melanocyte or goblet cell secretory lysosome targeting fusion moiety according to claim 1 or claim 2 wherein the polypeptide that specifically localizes to a secretory lysosome comprises a protease selected from the group consisting of: tryptases, chymases and carboxypeptidases.

4. A mast cell, basophil, hemopoietic cell, melanocyte or goblet cell secretory lysosome targeting fusion moiety according to any claim 1 to 3 wherein the polypeptide that specifically localizes to a mast cell, basophil, hemopoietic cell, melanocyte or goblet cell secretory lysosome is selected from the group consisting of: Mouse Mast Cell Protease -1, -2, -3. -4, -5, -6, and -7; Rat Mast Cell Protease I and Rat Mast Cell Protease II; human chymases; human tryptases; Cathepsin G-like protease; Cathepsin G; carboxypeptidase A; and hexosaminidase.

5. A mast cell, basophil, hemopoietic cell, melanocyte or goblet cell secretory lysosome targeting fusion moiety according to claim 4 wherein the protease is Rat Mast Cell Protease II according to GenBank accession no. J02712.

6. A cell comprising a mast cell, basophil, hemopoietic cell, melanocyte or goblet cell secretory lysosome targeting fusion moiety according to any claim 1 to 5.

7. A cell according to claim 6 wherein the fluorescent molecule is *Discosoma* sp. red fluorescent protein or green fluorescent protein.

8. A cell according to any claim 6 to 7, wherein the cell is selected from the group consisting of: mast cells, basophils, hemopoietic cells, melanocytes, and goblet cells.

9. A cell according to claim 8, wherein the cell is a mast cell.

10. A cell expressing a mast cell, basophil, hemopoietic cell, melanocyte or goblet cell secretory lysosome targeting fusion moiety according to claim 6 which is deposited with the American Type Culture Collection and assigned accession number PTA-4571.

11. A method for detecting and quantifying degranulation comprising: (a) incubating a cell expressing a mast cell, basophil, hemopoietic cell, melanocyte or goblet cell secretory lysosome targeting fusion moiety according to any one of claims 6 to 10 in the presence of a cell activator; (b) incubating the cell expressing the secretory lysosome targeting fusion moiety in the absence of the cell activator; and (c) detecting and quantifying the release of label in the supernatant in the presence of the cell activator compared to the release of label in the supernatant in the absence of the cell activator, wherein an increase in the release of label in the supernatant in the presence of the cell activator indicates degranulation.

12. A method for detecting and quantifying inhibition of degranulation comprising: (a) incubating a cell expressing a mast cell, basophil, hemopoietic cell, melanocyte or goblet cell secretory lysosome targeting fusion moiety according to any one of claims 6 to 10 with a cell activator in the presence of a test substance; (b) incubating the cell expressing the secretory lysosome targeting fusion moiety with the cell activator in the absence of the test substance; and (c) detecting and quantifying a change in the release of label in the supernatant in the presence of the test substance compared to the release of label in the supernatant in the absence of test substance, wherein a decrease in the release of label in the supernatant in the presence of test substance indicates inhibition of degranulation.

13. A method for detecting and quantifying degranulation at the single cell level comprising: (a) incubating a cell expressing a mast cell, basophil, hemopoietic cell, melanocyte or goblet cell secretory lysosome targeting fusion moiety according to any one of claims 6 to 10 in the absence of a cell activator; (b) detecting and quantifying the amount of label in the absence of a cell activator, (c) incubating the cell of step (a) in the presence of a cell activator; (d) detecting and quantifying the amount of label in the presence of the cell activator; and (e) detecting a change in the amount of label in the cell in the presence of the cell activator compared to the amount of label in the cell in the absence the cell activator, wherein a decrease in the amount of label indicates degranulation.

14. A method for detecting and quantifying degranulation at the single cell level comprising: (a) incubating a cell expressing a mast cell, basophil, hemopoietic cell, melanocyte or goblet cell secretory lysosome targeting fusion moiety according to any one of claims 6 to 10 in the presence of a cell activator; (b) detecting and quantifying the amount of label in the presence of the cell activator, (c) incubating the cell of step (a) in the presence of the cell activator and a test substance; (d) detecting and quantifying the amount of label in the presence of the cell activator and the test substance; and (e) comparing the amount of label in the cell in the presence of the test substance to the amount of label in the cell in the absence the test substance, wherein an increase in the amount of label in the presence of the test compound indicates degranulation.

15. The method according to any one of claims 11 to 14 wherein the cell activator is selected from the group consisting of: IgE and a multivalent antigen, phorbol myristate acetate, ionomycin, toll-like receptors, and protease receptors, preferably selected from the group consisting of: IgE and a multivalent antigen, phorbol myristate acetate, and ionomycin.

## Patentansprüche

1. Fusionseinheit, zielgerichtet auf sekretorische Lysosomen von Mastzellen, Basophilen, Hämatopoesezellen, Melanozyten oder Goblet-Zellen, umfassend: (a) ein Polypeptid, das sich spezifisch an einem sekretorischen Lysosom positioniert und nach Stimulation und Degranulation der Zelle mit dem Granulat-Gehalt in das extrazelluläre Medium freigesetzt wird, und (b) ein fluoreszierendes Markierungspolypeptid.

2. Fusionseinheit, zielgerichtet auf sekretorische Lysosomen von Mastzellen, Basophilen, Hämatopoesezellen, Melanozyten oder Goblet-Zellen, umfassend: (a) eine Nucleotidsequenz, die für ein Polypeptid kodiert, das sich spezifisch an einem sekretorischen Lysosom positioniert und nach Stimulation und Degranulation der Zelle mit dem Granulat-Gehalt in das extrazelluläre Medium freigesetzt wird, und (b) eine Nucleotidsequenz, die für ein fluoreszierendes Markierungspolypeptid kodiert.

3. Fusionseinheit, zielgerichtet auf sekretorische Lysosomen von Mastzellen, Basophilen, Hämatopoesezellen, Melanozyten oder Goblet-Zellen nach Anspruch 1 oder 2, wobei das Polypeptid, das sich spezifisch an einem sekretorischen Lysosom positioniert, eine Protease umfasst, die aus der Gruppe Tryptasen, Chymasen und Carboxypeptidasen ausgewählt ist.

4. Fusionseinheit, zielgerichtet auf sekretorische Lysosomen von Mastzellen, Basophilen, Hämatopoesezellen, Melanozyten oder Goblet-Zellen nach einem der Ansprüche 1 bis 3, wobei das Polypeptid, das sich spezifisch an einem sekretorischen Lysosom von Mastzellen, Basophilen, Hämatopoesezellen, Melanozyten oder Goblet-Zellen positioniert, aus der Gruppe ausgewählt ist, die besteht aus: Mäuse-Mastzellen-Protease-1, -2, -3, -4, -5, -6 und -7; Ratten-Mastzellen-Protease I und Ratten-Mastzellen-Protease II; humanen Chymasen; humanen Tryptasen; Cathepsin G-artiger Protease; Cathepsin G; Carboxypeptidase A; und Hexosaminidase.

5. Fusionseinheit, zielgerichtet auf sekretorische Lysosomen von Mastzellen, Basophilen, Hämatopoesezellen, Melanozyten oder Goblet-Zellen nach Anspruch 4, wobei es sich bei der Protease um Ratten-Mastzellen-Protease II gemäß Genbank-Hinterlegungsnummer J02712 handelt.

6. Zelle, umfassend eine Fusionseinheit, zielgerichtet auf sekretorische Lysosomen von Mastzellen, Basophilen, Hämatopoesezellen, Melanozyten oder Goblet-Zellen nach einem der Ansprüche 1 bis 5.

7. Zelle nach Anspruch 6, wobei es sich beim fluoreszierenden Molekül um rot fluoreszierendes Discosoma sp.-Protein oder grün fluoreszierendes Protein handelt.

8. Zelle nach einem der Ansprüche 6 bis 7, wobei die Zelle aus der Gruppe ausgewählt ist, die besteht aus Mastzellen, Basophilen-, Hämatopoesezellen, Melanozyten- und Goblet-Zellen.

9. Zelle nach Anspruch 8, wobei es sich bei der Zelle um eine Mastzelle handelt.

10. Zelle, die eine Fusionseinheit, zielgerichtet auf sekretorische Lysosomen von Mastzellen, Basophilen, Hämatopoesezellen, Melanozyten oder Goblet-Zellen nach Anspruch 6 exprimiert, hinterlegt bei der American Type Culture Collection unter der Hinterlegungsnummer PTA-4571.

11. Verfahren zum Nachweisen und quantitativen Bestimmen der Degranulation, umfassend: (a) das Inkubieren einer Zelle, die eine Fusionseinheit, zielgerichtet auf sekretorische Lysosomen von Mastzellen, Basophilen, Hämatopoesezellen, Melanozyten oder Goblet-Zellen nach einem der Ansprüche 6 bis 10 exprimiert, in Gegenwart eines Zellaktivators, (b) das Inkubieren der Zelle, die die Fusionseinheit, zielgerichtet auf sekretorische Lysosomen exprimiert, in Abwesenheit des Zellaktivators; und (c) das Nachweisen und quantitative Bestimmen der Freisetzung der Markierung im Überstand in Gegenwart des Zellaktivators, verglichen mit der Freisetzung der Markierung im Überstand in Abwesenheit des Zellaktivators, wobei eine Zunahme der Freisetzung der Markierung im Überstand in Gegenwart des Zellaktivators eine Degranulation anzeigt.

12. Verfahren zum Nachweisen und quantitativen Bestimmen der Hemmung der Degranulation, umfassend: (a) das Inkubieren einer Zelle, die eine Fusionseinheit, zielgerichtet auf sekretorische Lysosomen von Mastzellen, Basophilen, Hämatopoesezellen, Melanozyten oder Goblet-Zellen nach einem der Ansprüche 6 bis 10 exprimiert, mit einem Zellaktivator in Gegenwart einer Testsubstanz; (b) das Inkubieren der Zelle, die die sekretorische Lysosom-Ziel-Fusionseinheit exprimiert, mit dem Zellaktivator in Abwesenheit der Testsubstanz; und (c) das Nachweisen und quantitative Bestimmen einer Veränderung der Freisetzung der Markierung im Überstand in Gegenwart der Testsubstanz, verglichen mit der Freisetzung der Markierung im Überstand in Abwesenheit der Testsubstanz, wobei eine Abnahme der Freisetzung der Markierung im Überstand in Gegenwart der Testsubstanz eine Hemmung der Degranulation anzeigt.

13. Verfahren zum Nachweisen und quantitativen Bestimmen der Degranulation auf dem Einzelzellenniveau, umfassend: (a) das Inkubieren einer Zelle, die eine Fusionseinheit, zielgerichtet auf sekretorische Lysosomen von Mastzellen, Basophilen, Hämatopoesezellen, Melanozyten oder Goblet-Zellen nach einem der Ansprüche 6 bis 10 exprimiert, in Abwesenheit eines Zellaktivators; (b) das Nachweisen und quantitative Bestimmen der Menge der Markierung in Abwesenheit eines Zellaktivators; (c) das Inkubieren der Zelle von Stufe (a) in Gegenwart eines Zellaktivators; (d) das Nachweisen und quantitative Bestimmen der Menge der Markierung in Gegenwart des Zellaktivators; und (e) das Nachweisen einer Veränderung der Menge der Markierung in der Zelle in Gegenwart des Zellaktivators, verglichen mit der Menge der Markierung der Zelle in Abwesenheit des Zellaktivators, wobei eine Abnahme der Menge der Markierung eine Degranulation anzeigt.

14. Verfahren zum Nachweisen und quantitativen Bestimmen der Degranulation auf dem Einzelzellenniveau, umfassend: (a) das Inkubieren einer Fusionseinheit, zielgerichtet auf sekretorische Lysosomen von Mastzellen, Basophilen, Hämatopoesezellen, Melanozyten oder Goblet-Zellen nach einem der Ansprüche 6 bis 10 in Gegenwart eines Zellaktivators; (b) das Nachweisen und das quantitative Bestimmen der Menge der Markierung in Gegenwart des Zellaktivators; (c) das Inkubieren der Zelle von Stufe (a) in Gegenwart des Zellaktivators und einer Testsubstanz; (d) das Nachweisen und das quantitative Bestimmen der Menge der Markierung in Gegenwart des Zellaktivators und der Testsubstanz; und (e) das Vergleichen der Menge der Markierung in der Zelle in Gegenwart der Testsubstanz mit der Menge der Markierung in der Zelle in Abwesenheit der Testsubstanz, wobei ein Anstieg der Menge der Markierung in Gegenwart der Testverbindung eine Degranulation anzeigt.

15. Verfahren nach einem der Ansprüche 11 bis 14, wobei der Zellaktivator aus der Gruppe ausgewählt wird, die besteht aus: IgE und einem multivalenten Antigen, Phorbol-myristat-acetat, lonomycin, "toll"-artigen Rezeptoren und Protease-Rezeptoren, vorzugsweise ausgewählt aus der Gruppe, die besteht aus: IgE und ein multivalentes Antigen, Phorbol-myristat-acetat und lonomycin.

## Revendications

1. Entité de fusion ciblant les lysosomes sécrétoires de mastocytes, cellules basophiles, cellules hématopoïétiques, mélanocytes ou cellules caliciformes comprenant : (a) un polypeptide qui se positionne spécifiquement à un lysosome sécrétoire et est libéré avec le contenu des granules dans le milieu extracellulaire après stimulation et dégranulation de la cellule et (b) un polypeptide à marqueur fluorescent.

2. Entité de fusion ciblant les lysosomes sécrétoires de mastocytes, cellules basophiles, cellules hématopoïétiques, mélanocytes ou cellules caliciformes comprenant : (a) une séquence nucléotidique codant un polypeptide qui se positionne spécifiquement à un lysosome sécrétoire et est libéré avec le contenu des granules dans le milieu extracellulaire après stimulation et dégranulation de la cellule et (b) une séquence nucléotidique codant un polypeptide à marqueur fluorescent.

3. Entité de fusion ciblant les lysosomes sécrétoires de mastocytes, cellules basophiles, cellules hématopoïétiques, mélanocytes ou cellules caliciformes selon la revendication 1 ou la revendication 2 où le polypeptide qui se positionne spécifiquement à un lysosome sécrétoire comprend une protéase choisie dans le groupe consistant en : les tryptases, les chymases et les carboxypeptidases.

4. Entité de fusion ciblant les lysosomes sécrétoires de mastocytes, cellules basophiles, cellules hématopoïétiques, mélanocytes ou cellules caliciformes selon l'une quelconque des revendications 1 à 3 où le polypeptide qui se positionne spécifiquement à un lysosome sécrétoire de mastocytes, cellules basophiles, cellules hématopoïétiques, mélanocytes ou cellules caliciformes est choisi dans le groupe consistant en : la protéase de mastocyte de souris -1, -2, -3, -4, -5, -6 et -7 ; la protéase de mastocyte de rat I et la protéase de mastocyte de rat II ; les chymases humaines ; les tryptases humaines ; la protéase analogue à la cathepsine G ; la cathepsine G ; la carboxypeptidase A ; et l'hexosaminidase.

5. Entité de fusion ciblant les lysosomes sécrétoires de mastocytes, cellules basophiles, cellules hématopoïétiques, mélanocytes ou cellules caliciformes selon la revendication 4 où la protéase est la protéase de mastocyte de rat II selon GenBank n° d'ordre J02712.

6. Cellule comprenant une entité de fusion ciblant les lysosomes sécrétoires de mastocytes, cellules basophiles, cellules hématopoïétiques, mélanocytes ou cellules caliciformes selon l'une quelconque des revendications 1 à 5.

7. Cellule selon la revendication 6 où la molécule fluorescente est la protéine fluorescente rouge ou la protéine fluorescente verte de *Discosoma* sp.

8. Cellule selon l'une quelconque des revendications 6 à 7 où la cellule est choisie dans le groupe consistant en : les mastocytes, les cellules basophiles, les cellules hématopoïétiques, les mélanocytes et les cellules caliciformes.

9. Cellule selon la revendication 8 où la cellule est un mastocyte.

10. Cellule exprimant une entité de fusion ciblant les lysosomes sécrétoires de mastocytes, cellules basophiles, cellules hématopoïétiques, mélanocytes ou cellules caliciformes selon la revendication 6 qui est déposée auprès de la American Type Culture Collection et à laquelle est attribué le numéro d'ordre PTA-4571.

11. Procédé pour détecter et quantifier la dégranulation comprenant : (a) incuber une cellule exprimant une entité de fusion ciblant les lysosomes sécrétoires de mastocytes, cellules basophiles, cellules hématopoïétiques, mélanocytes ou cellules caliciformes selon l'une quelconque des revendications 6 à 10 en présence d'un activateur cellulaire ; (b) incuber la cellule exprimant l'entité de fusion ciblant les lysosomes sécrétoires en l'absence de l'activateur cellulaire ; et (c) détecter et quantifier la libération de marqueur dans le surnageant en présence de l'activateur cellulaire comparée à la libération de marqueur dans le surnageant en l'absence de l'activateur cellulaire, où une augmentation de la libération de marqueur dans le surnageant en présence de l'activateur cellulaire indique la dégranulation.

12. Procédé pour détecter et quantifier une inhibition de la dégranulation comprenant : (a) incuber une cellule exprimant une entité de fusion ciblant les lysosomes sécrétoires de mastocytes, cellules basophiles, cellules hématopoïétiques, mélanocytes ou cellules caliciformes selon l'une quelconque des revendications 6 à 10 avec un activateur cellulaire en présence d'une substance test ; (b) incuber la cellule exprimant l'entité de fusion ciblant les lysosomes sécrétoires avec l'activateur cellulaire en l'absence de la substance test; et (c) détecter et quantifier un changement dans la libération de marqueur dans le surnageant en présence de la substance test comparée à la libération de marqueur dans le surnageant en l'absence de substance test, où une diminution de la libération de marqueur dans le surnageant en présence de substance test indique une inhibition de la dégranulation.

13. Procédé pour détecter et quantifier la dégranulation au niveau d'une seule cellule comprenant : (a) incuber une cellule exprimant une entité de fusion ciblant les lysosomes sécrétoires de mastocytes, cellules basophiles, cellules hématopoïétiques, mélanocytes ou cellules caliciformes selon l'une quelconque des revendications 6 à 10 en l'absence d'un activateur cellulaire ; (b) détecter et quantifier la quantité de marqueur en l'absence d'un activateur cellulaire, (c) incuber la cellule de l'étape (a) en présence d'un activateur cellulaire ; (d) détecter et quantifier la quantité de marqueur en présence de l'activateur cellulaire ; et (e) détecter un changement dans la quantité de marqueur dans la cellule en présence de l'activateur cellulaire comparée à la quantité de marqueur dans la cellule en l'absence de l'activateur cellulaire, où une diminution de la quantité de marqueur indique la dégranulation.

14. Procédé pour détecter et quantifier la dégranulation au niveau d'une seule cellule comprenant : (a) incuber une cellule exprimant une entité de fusion ciblant les lysosomes sécrétoires de mastocytes, cellules basophiles, cellules hématopoïétiques, mélanocytes ou cellules caliciformes selon l'une quelconque des revendications 6 à 10 en présence d'un activateur cellulaire ; (b) détecter et quantifier la quantité de marqueur en présence de l'activateur cellulaire ; (c) incuber la cellule de l'étape (a) en présence de l'activateur cellulaire et d'une substance test ; (d) détecter et quantifier la quantité de marqueur en présence de l'activateur cellulaire et de la substance test ; et (e) comparer la quantité de marqueur dans la cellule en présence de la substance test à la quantité de marqueur dans la cellule en l'absence de la substance test, où une augmentation de la quantité de marqueur en présence du composé test indique la dégranulation.

15. Procédé selon l'une quelconque des revendications 11 à 14 où l'activateur cellulaire est choisi dans le groupe consistant en : IgE et un antigène multivalent, myristate acétate de phorbol, ionomycine, récepteurs analogues à toll, et récepteurs de protéases, de préférence choisi dans le groupe consistant en : IgE et un antigène multivalent, myristate acétate de phorbol et ionomycine.


### SEQUENCE LISTING

<110> Boehringer Ingelheim Pharmaceuticals, Inc.
<120> METHODS FOR TARGETING SECRETORY LYSOSOMES
<130> 9/251 PCT
<140> To be assigned
   <141> 2003-08-08
<150> US 60/403,464
   <151> 2001-08-14
<160> 2
<170> PatentIn version 3.1
<210> 1
   <211> 33
   <212> DNA
   <213> Rattus norvegicus
<400> 1
   tcagatctcg agatgcaggc cctactattc ctg 33
<210> 2
   <211> 35
   <212> DNA
   <213> Rattus norvegicus
<400> 2
   ctgcagaatt cggctacttg tattaatgac tgcat 35